# FASCICULE DE BREVET EUROPEEN

(11) **EP 4 154 814 B1**
(45) Date de publication et mention de la délivrance du brevet: **03.12.2025**
(21) Numéro de dépôt: 22207532.7
(22) Date de dépôt: 02.08.2019
(51) Int. Cl.: A61B 5/15, A61B 5/00, A61B 5/157, A61B 5/145

(54) **SYSTEME DE SURVEILLANCE CORPORELLE**
KÖRPERÜBERWACHUNGSSYSTEM
BODY MONITORING SYSTEM

(30) Priorité: 03.08.2018 EP 18306070
(43) Date de publication de la demande: 29.03.2023
(62) Demande divisionnaire de: 19748828.1
(73) Titulaire: WIZP AS, 3125 Tønsberg (NO)
(72) Inventeur: PIERART, Luc, 94800 Villejuif (FR)
(74) Mandataire: Regimbeau

(56) Documents cités:
- EP-A2- 2 298 177
- WO-A1-2016/140760
- CN-A- 108 310 615
- US-A1- 2003 153 900

## Description

### DOMAINE TECHNIQUE GENERAL

La présente invention se rapporte un système de surveillance corporelle via analyse de liquide corporel, typiquement interstitiel, à l'aide de microaiguilles.

Plus précisément, la présente invention concerne un patch pour la gestion du maintien des microaiguilles dans la peau.

### ETAT DE L'ART

Certaines pathologies comme le diabète nécessitent une surveillance quotidienne de paramètres biochimiques du corps humain, i.e. des concentrations en certains composés (la glycémie dans l'exemple du glucose).

Pour cela, il est courant de piquer un point de la peau de sorte à faire perler une goutte de sang, et d'analyser cette goutte soit de façon réactive (par exemple avec une bandelette), soit de façon électronique (par exemple par au moins d'un capteur analytique), de façon à estimer le ou les paramètres cible.

On connait aujourd'hui des systèmes évolués bien moins invasifs qui se contentent d'analyser le liquide interstitiel, c'est-à-dire le fluide qui remplit l'espace entre les capillaires sanguins et les cellules. Il a en effet une composition ionique proche de celle du plasma sanguin.

Ces systèmes évolués permettent ainsi de surveiller les paramètres biochimiques souhaités de façon transcutanée, c'est-à-dire sans nécessité de percer régulièrement la peau et de prélever.

On connait des dispositifs avec microaiguilles, qui ont l'avantage d'être moins invasive que des aiguilles classiques. Toutefois, il est important que ces microaiguilles restent en place.

Il existe pour cela des dispositifs à demeure, où des microaiguilles sont maintenues sur la peau avec une bande adhésive. Toutefois, on souhaite pouvoir effectuer un contrôle en continu ou quasi-continu, ce qui requiert des dispositifs autonomes. On pourra citer le dispositif GlucoWatch, qui utilisait l'iontophorèse (et non pas des aiguilles).

WO 2016140760 décrit un système de surveillance corporelle destiné à être attaché à un membre comprenant un patch et une capsule positionnés dans boitier maintenu en place sur le membre par une lanière.

On connait aussi le dispositif du document WO2018104647, qui présente un boitier comprenant une capsule amovible, la capsule accueillant des microaiguilles configurées pour prélever du liquide interstitiel. Le boitier, quant à lui, accueille la majeure partie de l'électronique.

Ce dispositif portatif, typiquement au poignet, permet une mesure en continu et il suffit de changer la capsule pour changer de microaiguilles.

Toutefois, lorsqu'un tel dispositif est porté au poignet, la difficulté réside dans la conciliation d'un système permettant maintien des microaiguilles en position dans la peau et d'un système remplaçable.

En outre, il est important que le dispositif soit facilement utilisable.

L'invention vise à adresser ces difficultés.

### PRESENTATION DE L'INVENTION

Pour répondre à certaines de ces problématiques, l'invention propose un système selon la revendication 1.

Le système de l'invention est avantageusement complété par les caractéristiques des revendications 2 à 8, prises seules ou en une quelconque de leur combinaison techniquement possible

Dans un mode de réalisation, la zone rigide présente une ligne de sécabilité, interrompue par l'orifice, la ligne de sécabilité étant configurée pour permettre de plier la zone rigide du patch.

Dans un mode de réalisation, la ligne de sécabilité est un axe de symétrie de l'orifice.

Dans un mode de réalisation, l'orifice comprend sur une paroi interne un sillon ou des renfoncements ponctuels, configurés pour recevoir une capsule.

Dans un mode de réalisation, la zone rigide présente une dureté shore plus importante que la zone souple, par exemple une dureté shore comprise entre 20 et 40 pour la zone souple et une dureté shore comprise entre 60 et 80 pour la zone rigide.

Dans un mode de réalisation, le matériau de la zone rigide est plus dur que le matériau de la zone souple, les deux matériaux étant différents.

Dans un mode de réalisation, la zone rigide est au moins deux fois plus épaisse que la zone souple, préférablement au moins trois fois plus épaisse.

Dans un mode de réalisation, le patch comprend un adhésif plan recouvrant substantiellement une face plane des zones rigides et souples.

Dans un mode de réalisation, l'adhésif est à base soit de silicone, soit d'acrylique.

Dans un mode de réalisation, le corps principal, préférentiellement la zone souple, comprend une pluralité d'ouvertures traversantes.

Dans un mode de réalisation, le corps principal, préférentiellement la zone souple, comprend une pluralité d'ouvertures traversantes, et l'adhésif comprend une pluralité d'ouvertures traversantes, la taille des ouvertures du corps principal étant supérieure à la taille des ouvertures de l'adhésif, et/ou la densité de surface d'ouverture du corps principal est supérieure à la densité de surface d'ouverture de l'adhésif, et/ou la densité d'ouvertures du corps principal est supérieure à la densité d'ouvertures de l'adhésif.

Dans un mode de réalisation, les ouvertures du corps principal sont en bais.

Dans un mode de réalisation, la zone rigide comprend un décrochement présentant une épaisseur moindre que l'épaisseur du reste de la zone rigide.

Dans un mode de réalisation, la capsule a une épaisseur identique à l'épaisseur de la zone rigide du corps principal du patch.

### PRESENTATION DES FIGURES

D'autres caractéristiques et avantages de la présente invention apparaîtront à la lecture de la description qui va suivre d'un mode de réalisation préférentiel. Cette description sera donnée en référence aux dessins annexés dans lesquels :
- La figure 1 illustre en vue éclatée un bracelet, un boitier, une capsule et un patch tel qu'utilisable dans le cadre de l'invention,
- Les figure 2a et 2b illustrent une vue en trois dimension, pleine et coupée, d'un patch et d'une capsule attachée au patch,
- La figure 3 illustre une vue en coupe des figures 2a et 2b,
- La figure 4 illustre l'agencement des différents modules qui forment le système (dimensions différentes des dimensions des figures 2a et 2b).

### DESCRIPTION DETAILLEE

### Architecture générale

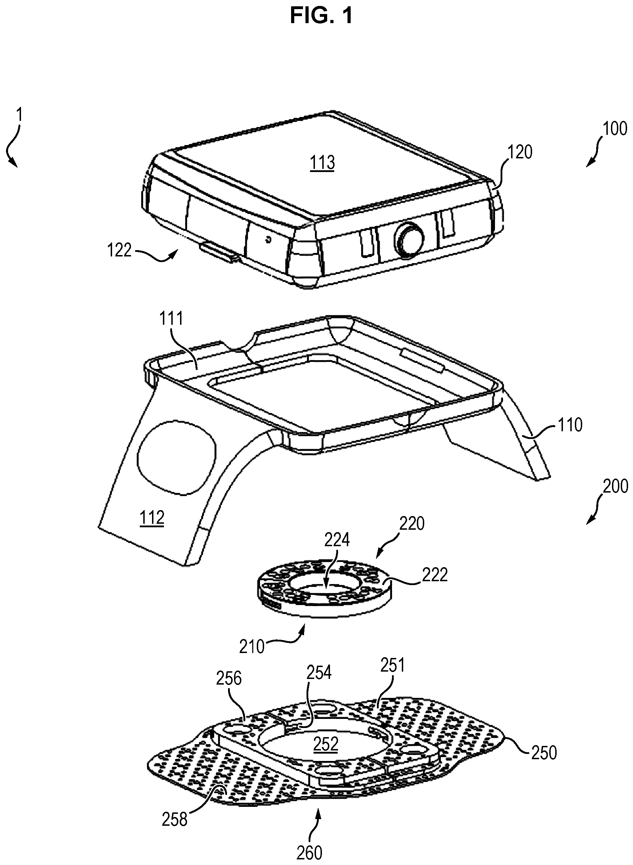

En référence aux **figures 1 à 4****,** la présente invention concerne un système électronique 1 de surveillance corporelle. Il s'agit d'une amélioration du dispositif du document WO2018104647. Par conséquent, l'invention se place dans le même concept général de système intégral autonome à faible douleur, faible risque hygiénique et réutilisable.

Par surveillance corporelle, on entend la vérification de constantes biochimiques d'une personne porteuse du système 1, typiquement la concentration en une protéine, une hormone, un marqueur, en oxygène, en nutriments, etc., dans le liquide interstitiel de la personne. On citera l'exemple de la glycémie. L'homme du métier pourra surveiller si besoin d'autres grandeurs physiques corporelles telles que le lactate, l'hydratation, etc.

La description sera illustrée avec du liquide interstitiel mais s'applique aux autres liquides corporels tels que le sang.

Le système 1 est dit autonome, car il ne nécessite pas l'utilisation de matériel supplémentaire.

Le système 1 est destiné à être attaché à un membre d'un être vivant, typiquement un bras ou une jambe d'un être humain. La zone d'attache privilégiée est le poignet où le système 1 s'apparente à une montre.

Le système 1 est formé de deux modules 100, 200 **(****figure 1****)** reliées entre eux par une liaison séparable 300 qui est réutilisable. On définit ainsi une position couplée et une position libre. En position couplée, les deux modules 100, 200 ne sont pas séparés physiquement et peuvent échanger des données.

Le premier module 100 comprend notamment des moyens d'attache et de serrage 110 du système 1 à un membre (qu'on appellera lanière 112 ou bracelet) et le deuxième module 200 comprend une capsule 220 qui intègrent des microaiguilles 210 configurées pour être insérées dans la peau (dans une partie superficielle de l'épiderme). Ces microaiguilles 210, lorsque le premier module 100 est en position sur le membre, permettent de prélever et/ou d'analyser un fluide corporel, comme mentionné précédemment.

Les microaiguilles consistent avantageusement en un réseau de microaiguilles 210 au contact de la peau lorsque la capsule 220 est placée sur le corps d'une personne. Les microaiguilles 210 peuvent donc être soit creuses, pour prélever du liquide, soit pleines, pour analyser directement le liquide. Dans le premier cas, typiquement, les microaiguilles 210 permettent l'extraction de liquide interstitiel du derme de façon indolore sans perlement de sang, et l'envoie vers un capteur dans le système 1 (plus précisément préférablement dans le deuxième module 200). Dans le deuxième cas, les microaiguilles 210 ne prélèvent pas de fluide et intègrent le capteur sur leur surface, sous la forme d'un matériau biochimique apte à réagir avec l'analyte que l'on souhaite mesurer dans le fluide. Dans le cas de microaiguilles percées formant un canal dans chaque microaiguille, un prélèvement peut être réalisé en connectant de manière fluidique un système de pompage du fluide interstitiel au canal, ou simplement par capillarité. Un système d'analyse peut comprendre des microaiguilles chacune pourvue d'une électrode ou d'un ensemble d'électrodes, ou être déporté après les microaiguilles, de sorte à entraîner une réaction électrochimique adaptée à détecter un analyte dans le fluide interstitiel.

De façon préférée, lesdits microaiguilles comprennent entre quatre et cinquante microaiguilles, sensiblement pyramidales, avec des pointes d'une hauteur comprise entre 100µm et 1000µm, préférablement 0.3mm et 0.8mm. Chacune de ces caractéristiques avantageuses des microaiguilles 210 peut être prise séparément ou en combinaison avec les autres.

Les deux modules 100 et 200 présentent chacun une face de couplage 122, 222, de forme complémentaire, qui permet de placer le deuxième module 200 dans un emplacement d'accueil du premier module 100

Enfin, le premier module 100 et le deuxième module 200 sont configurés pour être couplés par une liaison séparable 300.

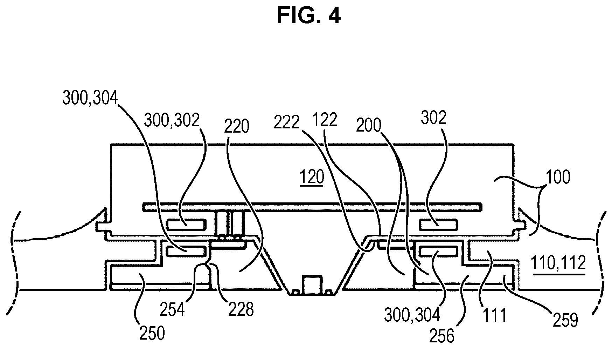

Comme illustré sur les **figures 1** **et** **4****,** le premier module 100 comprend en outre un boitier 120 dans lequel sont disposés des moyens de traitement de données (en particulier un processeur ou un microcontrôleur) configurés pour traiter des mesures acquises par le capteur, et le cas échéant des moyens de stockage de données (notamment une mémoire, en particulier de type flash, et/ou la mémoire du microcontrôleur) permettant par exemple de stocker ces mesures, et/ou une date de la première utilisation de chaque capteur pour calculer une date de péremption du ou des capteurs (les capteurs biochimiques ont une durée de vie limitée). Les moyens de traitement de données servent aussi à générer des consignes vers différentes composants. Dans le cadre de cette description, ces différentes fonctions sont assurées par une même unité. Toutefois, il est possible de prévoir des processeurs dédiés. Le système comprend également une batterie pour l'alimentation électrique des composants, avantageusement rechargeable, par exemple via un port (dont on comprend qu'il peut également servir à connecter le système 1 par exemple à un ordinateur pour télécharger les données acquises et/ou traitées).

De façon préférée le système 1 peut comprendre des moyens de connexion sans fil (en particulier de type WiFi, mais également Bluetooth, voire 3G/4G) pour connexion à un réseau, en particulier internet, et une interface utilisateur tel qu'un écran 113, éventuellement tactile pour afficher les résultats de la surveillance à l'utilisateur.

L'homme du métier connait des algorithmes de traitement de mesures de capteurs 24 et des interfaces associées, et saura les implémenter dans le présent système 1.

Le boitier 120 comprend en outre des connecteurs électriques, sur sa face de couplage 122, qui se couple avec la face de couplage 222 de la capsule 220.

La capsule 220 du deuxième module 200 a une forme de boite fermée, typiquement étanche, qui peut se coupler avec le boitier 120. Cette capsule 220 est interchangeable, ce qui permet d'obtenir un système économique et efficace, où seules les parties dites consommables doivent être changées. La capsule 220 peut avoir une forme annulaire, avec une ouverture traversante 224 au centre. Dans une variante mentionnée précédemment, le capteur est positionné à l'intérieur de la capsule 220 (ou alors dans le boitier 120) et analyse le fluide prélevé par les microaiguilles. La capsule 220 comprend des connecteurs électriques 226, sur une face de couplage 222 avec le boitier 120, qui peuvent coopèrent avec les connecteurs électriques du boitier 120. S'il y a transmission de fluide depuis le deuxième module vers le premier alors des connecteurs fluidiques complémentaires sont prévus sur la capsule 220 et le boitier 120.

Enfin, le deuxième module 200 comprend un patch 250, solidaire de façon amovible à la capsule 220, qui est décrit en détail ci-dessous.

Le deuxième module 200 (capsule 220 et patch 250) forme un ensemble interchangeable du système qui est choisi selon le type de surveillance voulu et en fonction de l'état de détérioration des microaiguilles 210 et/ou du capteur.

En effet, dans la mesure où la capsule 220 contient les microaiguilles 210 et/ou le capteur (notamment dans le cas de microaiguilles qui prélèvent), changer de capsule 220 permet de changer de matériel si celui-ci est en fin de vie ou si l'on souhaite changer de grandeur physique mesurée, en une manipulation simple, rapide et sure, sans devoir jeter d'autres parties (en particulier le premier module 100).

Et dans la mesure où la capsule 220 minimise la quantité d'élément et/ou matériaux coûteux (équipement électronique avancé tel qu'une batterie ou des moyens de communication sans fil), elle est relativement peu chère.

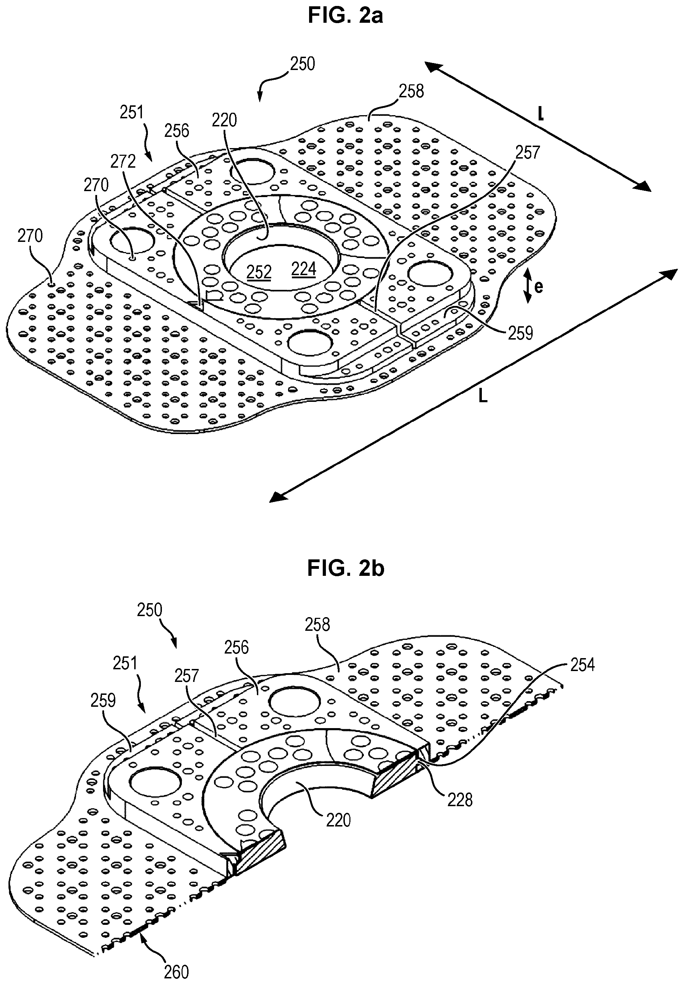

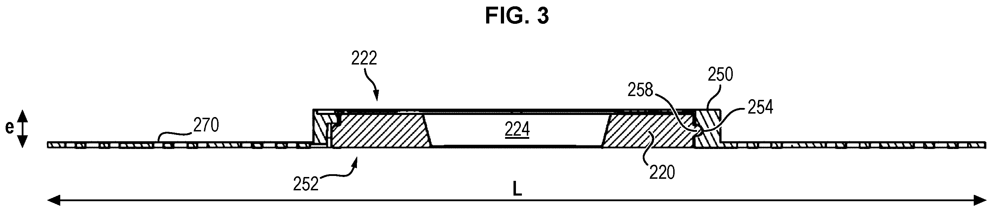

### Le patch 250 (figures 2a, 2b et 3)

Le patch 250 fonctionne comme un adhésif pour maintenir les microaiguilles 210 enfoncées dans la peau. La capsule 220 étant attachée au patch 250, elle profite du pouvoir adhésif du patch 250 pour être maintenue contre la peau.

Le patch 250, peu coûteux, peut être changé plus fréquemment que la capsule 220. Cela est rendu possible grâce à la fixation amovible entre le patch 250 et la capsule 220.

Il est conçu pour ressembler à un pansement, afin que les personnes qui le portent ne se sentent pas stigmatisés par le port d'un élément inhabituel.

Le patch 250 a une longueur L comprise entre 3cm et 10cm, une largeur I comprise entre 3cm et 10cm et une épaisseur e comprise entre 0,1cm et 1cm.

Le patch 250 comprend un corps principal 251 avec un orifice, dit orifice central 252, configuré pour recevoir la capsule 220, et avantageusement un adhésif 260 (si le ou les matériaux du patch ne fonctionnent pas déjà comme un adhésif par exemple). Par central, on entend que l'orifice central 252 se trouve intégralement au sein du corps principal 251, et non pas sur un bord. Néanmoins, « central » ne signifie pas nécessairement « centré » : il se peut que l'orifice central 252 ne soit pas centré sur le corps principal 251.

L'orifice 252 fait au moins 2mm de diamètre (ce n'est donc pas un micro-orifice qui permet d'aérer le patch).

Le corps principal 251 comprend deux zones : une zone rigide 256, qui s'étend autour de l'orifice central 252, et une zone souple 258, qui s'étend au moins de part et d'autre (préférablement tout autour) de la zone rigide 256. La zone souple 258 s'étend essentiellement selon deux côtés opposés de la zone rigide 256 dans le sens de la longueur L, pour épouser la courbure du membre. Mais préférablement, la zone souple 258 trouve tout autour de la zone rigide 256, avec une longueur plus importante dans le sens de la longueur L (entre 1 et 3cm par exemple) que dans le sens de la largeur (entre 0,2 et 0,5 cm par exemple).

Les zones rigides 256 et souple 258 correspondent à différentes portions du patch.

La zone rigide 256 est solide, difficilement pliable lors d'une utilisation normale avec des doigts. Inversement, la zone souple 258 est pliable sans forcer, presque sous son propre poids.

Les zones rigide 256 et souple 258 ne présentent pas la même épaisseur. Préférablement, la zone rigide 256 est deux fois plus épaisse, voire trois fois plus épaisse que la zone souple 258. La zone souple 258 quant à elle est la plus fine possible, pour deux raisons : l'adhérence et l'absorption des chocs.

En effet, à l'instar d'un pansement, la souplesse permet d'épouser le contour du membre et en particulier du poignet, ce qui assure une bonne adhérence.

De plus, pour éviter l'arrachement involontaire des microaiguilles 210 de la peau, il est important que les efforts d'arrachement qui s'exercent sur le patch 250 ne soit pas transmis à la capsule 220. A cet égard, la souplesse de la zone souple 258 assure une absence de transmission de couple ou de force à la zone rigide 256 et donc à la capsule 220. La faible épaisseur de la zone souple 258 la rend aussi invisible en utilisation et donc peu encline à être arrachée par erreur.

Inversement, la zone rigide 258 a pour fonction de tenir la capsule 220 en place et de lui transmettre les efforts de maintien qu'elle reçoit depuis le deuxième module 200, qui appuie sur la zone rigide en direction de la peau.

Le patch 250, avec la zone rigide et la zone souple, remplit ainsi deux fonctions importantes du patch 250.

Vu de profil **(****figure 3****),** le patch 250 présente une face plane, configurée pour être au contact de la peau, et une face non plane, à cause de la zone rigide 258, configurée pour être au contact de premier module 100.

La zone rigide 256 a une forme, dans le plan, similaire à la forme du boitier 120, de sorte que lorsque le système 1 est porté, la zone rigide 256 est masquée par le boitier 120 et le bracelet 110. Sur les **figures,** cette forme est rectangulaire avec des angles arrondis.

Les zones rigide 256 et souple 258 sont typiquement réalisées dans deux matériaux (par exemple polymères) aux caractéristiques physiques différentes. La dureté shore de la zone rigide 256 est par exemple comprise entre 40 et 60, tandis que la dureté shore de la zone souple 258 est par exemple comprise entre 20 et 40. La différence de rigidité s'obtient soit par une épaisseur différente, soit par un choix de matériau différent, soir par un même matériau avec des propriétés différentes, ou un mélange de ces deux critères.

Par exemple, la zone souple 258 est en silicone et la partie rigide est dans un autre plastique (pièce rapportée).

Par exemple la zone rigide 256 est aussi en silicone, plus rigide.

Dans un mode de réalisation, une pièce rigide est rapportée sur une couche souple pour former la zone rigide. Dans un autre mode de réalisation, la couche souple est rapportée autour d'une pièce rigide.

### Le patch 250 et la capsule 220, sécabilité du patch 250.

Autour de l'ouverture 252, l'épaisseur de la zone rigide 256 est sensiblement égale (voir égale) à l'épaisseur de la capsule 220 (à plus ou moins 5% de l'épaisseur de la capsule 210). La zone souple est plus fine pour pouvoir épouser la forme du membre et pour aussi gêner le moins possible l'utilisateur dans sa vie quotidienne. En effet, dans le cas d'un poignet, la capsule 220 est positionnée sur une région plutôt plate du membre, alors que la zone souple 258 du patch 250 s'étend jusqu'au courbure du poignet.

Pour solidariser la capsule 220 au patch 250 (ou l'inverse), l'orifice central 252 comprend, sur sa paroi interne, un sillon ou des renfoncement ponctuels 254. Ils fonctionnent comme des encoches pour recevoir une protubérance 228 sur une paroi annulaire extérieur de la capsule 220. Préférablement, l'agencement des encoches 254 et des protubérances 228 est tel qu'il empêche la rotation de la capsule 220 au sein de l'orifice central 252 du patch 250. Une solution préférentielle consiste à prévoir une ou plusieurs encoches ponctuelles, qui bloquent la rotation.

En outre, la rigidité de la zone rigide 258 permet de rendre le patch 250 sécable de part et d'autre de l'orifice central 252, pour faciliter l'insertion de la capsule 220 : il suffit d'insérer la capsule 220 contre une partie de l'orifice central 252, puis de déplier le patch 250 pour le remettre à plat. A cet égard, un choix adapté de matériau pour la zone rigide 256 peut suffire mais il y a un risque que la sécabilité ne se fasse pas au bon endroit. Pour remédier à ce problème, la zone rigide 256 comprend une ligne de sécabilité 257 qui s'étend sur toute la zone rigide 256 (sur les figures : dans le sens de la largeur I), en passant par l'orifice central 252. Cette ligne de sécabilité 257 facilite la rupture du patch 250 pour l'insertion de la capsule 220, ce qui facilite la manipulation. Préférablement, la ligne de sécabilité 257 correspond à un axe de symétrie de l'ouverture 252 (aux encoches 258 près), qui est généralement un axe de symétrie de la zone rigide 256 et du corps principal 251. La ligne de sécabilité 257 est typiquement réalisée par un sillon sur la surface de la zone rigide 256.

Du fait de la mise en place de capsule 220 par pliage de l'orifice central 252, les renfoncements ponctuels de la paroi interne de l'orifice central 252 ne sont pas disposés de façon symétrique par rapport à la ligne de sécabilité 257 : en effet, la mise en place du patch 250 se fait par insertion sur une moitié de l'orifice central 252 plié. Par conséquent, la disposition des sillons ou des renfoncements ponctuels est adaptée en conséquence.

### Le patch 250 et la lanière/bracelet 110

Lorsque les deux modules 100, 200 sont assemblés, le boitier 120 et le patch 250 sont en contact avec la zone rigide 256 qui est autour de l'ouverture 252 (voir **figure 4****).** Cela permet une première reprise d'effort vers la capsule 220, via le patch 250.

Plus précisément, selon le mode de réalisation illustré sur les **figures 1****,** **2a** **et** **3****,** afin d'avoir aussi le bracelet ou la lanière 210 qui exerce un effort sur le patch 250 (pour le plaquer contre la peau), la zone rigide 256 comprend, à ses extrémités latérales un décrochement 259 de la zone rigide 256 qui se manifeste par une épaisseur moindre (mais typiquement toujours supérieure à celle de la zone souple 258). Complémentairement, le bracelet 110 comprend, de chaque côté (préférablement sur les quatre côtés dans le cas d'une forme rectangulaire ou sur toute la périphérie dans le cas d'une forme circulaire ou ovale), une languette 111 qui se positionne au-dessus du décrochement 259 (et sous le boitier 120). La languette 111 et le décrochement 259 ont une largeur faible, de quelques millimètres, mais s'étendent sur toute une longueur de la zone rigide 256.

Dans la présente description, sauf mention inverse, les références à l'épaisseur de la zone rigide 256 concernent l'épaisseur en l'absence de décrochement. On peut ainsi définir une région principale de la zone rigide 256, qui possède l'épaisseur maximum (sensiblement égale à celle de la capsule) et une région secondaire de la zone rigide 258, qui correspond au décrochement 259, qui possède une épaisseur moindre.

La reprise d'effort par le bracelet 110 présente deux intérêts : un premier intérêt est que l'effort vient du bracelet 110 lui-même, que l'on serre sur son poignet ou son membre (transmission directe), et un deuxième intérêt vient du fait que le système 1 peut être porté sans boitier 120. Dès lors, pour bien tenir le patch 250, l'effort doit être repris par le bracelet 110 et non le boitier 120.

### Adhésif

L'adhésif 260 est sous forme d'une couche plane, en regard de faces planes des zones rigide 256 et souple 254, du côté destiné à être sur la peau.

L'adhésif 260 utilisé est avantageusement à base de silicone pour sa capacité à pouvoir être décollé et recollé, pour les faibles irritations qu'il provoque, pour la meilleure gestion des douches et l'absence de trace sur la peau. De plus, le silicone fonctionne très bien en arrachement latéral et peu en traction, mais il s'agit du bracelet/lanière qui assure cette fonction. On peut alternativement utiliser un adhésif à base d'acrylique, qui possède un pouvoir adhérant plus fort que le silicone mais qui n'est pas décollable et recollable.

Pour protéger l'adhésif du patch 250 (conditionnement, transport, etc.), l'adhésif 260 peut être recouvert d'une couche de papier pelable.

### Respirabilité

Pour faciliter la respiration de la peau et éviter les effets de macération, le corps principal 251 comprend une pluralité d'ouvertures traversantes 270, à la fois dans la zone souple et dans la zone rigide. Les ouvertures ont un diamètre compris entre 0,1 et 2 mm. De la même façon, l'adhésif 260 comprend des ouvertures traversantes (non visible sur les figures), pour que la communication d'air se fasse depuis la peau.

Pour s'assurer que les ouvertures de l'adhésif 260 communiquent avec des ouvertures 270 du corps principal 251, la densité des ouvertures de l'adhésif est supérieure à celle des ouvertures 270 du corps principal 251 et la taille des ouvertures de l'adhésif est inférieure à celle des ouvertures 270 du corps principal 251. Ainsi, toute superposition de l'adhésif sur le corps principal génère une communication entre les ouvertures, sans qu'il ne soit nécessaire de chercher à aligner des ouvertures entre elles.

Enfin, pour des questions d'esthétisme (absence de visibilité de la peau quand le patch est porté seul), les ouvertures du corps principal peuvent être inclinées (par rapport à un plan formé par le patch 250 quand il est mis à plat).

### Marqueur

Enfin, le patch 250 comprend un signe distinctif 272 aux abords de l'ouverture afin de pouvoir orienter aisément la capsule 220 dans le patch 250. Ce signe distinctif est représenté par une flèche 272 sur les figures. Deux flèches 272 peuvent être prévues, une de chaque côté, en fonction du sens de mise en place de la capsule 220. Un signe similaire peut être présent sur la capsule 220. Les signes entre le patch 250 et la capsule 220 sont préférablement identiques ou symétriques.

Les signes distinctifs 272 peuvent se retrouver sous le patch 250, côté peau. De la même façon, les signes entre le patch 250 et la capsule 220 sont préférablement identiques ou symétriques.

Le sens de la flèche peut changer entre les deux faces du patch pour que visuellement l'utilisateur mettre la capsule dans le bon sens (ie. mettre les microaiguilles côté peau du patch).

### Fixation

Pour pouvoir attacher le patch 250 au boitier 120, des aimants ou matériaux ferromagnétiques 304 peuvent être situés dans la zone rigide 256, de préférence de façon régulière autour de l'ouverture 252.

### Kit de patchs

Du fait de sa fonction, le patch 250 est amené à être changé plus souvent que la capsule. Par conséquent, il peut être fourni en plusieurs exemplaires, tous identiques. Alternativement, comme le patch 250 doit être adapté aux circonstances (type de peau, taille du membre, conditions d'utilisation), la largeur et/ou la longueur et/ou les propriétés de l'adhésif peuvent être différentes entre deux patchs du kit.

De plus, différentes capsules peuvent être utilisées, avec des formes différentes: les patchs peuvent donc avoir des formes de l'orifice 252 différentes.

## Revendications

1. Système de surveillance corporelle (1) destiné à être attaché à un membre, comprenant :
- un patch (250) comprenant un corps principal (251) avec un orifice (252) d'au moins 2mm de diamètre, le corps principal (251) comprenant une zone souple (258) sur laquelle est rapportée une zone rigide (256) la zone rigide (256) étant disposée autour de l'orifice (252);
- une capsule (220), la capsule (220) étant configurée pour se placer dans l'ouverture du patch (250) et être solidaire de celui-ci, la capsule (220) comprenant des microaiguilles (210) configurées pour être insérées dans la peau pour prélever ou analyser un fluide corporel du porteur du système de surveillance corporelle (1) lorsque ce dernier est positionné sur un membre d'un utilisateur ;
- un boitier (120), apte à être couplé avec la capsule (220), à l'intérieur duquel se trouve une batterie et un processeur, le processeur étant configuré pour traiter des données obtenues à l'aide du prélèvement ou de la mesure faite par les microaiguilles (210) ;
- une lanière, configuré pour maintenir le boitier (120) en place sur le membre, le système étant tel que la capsule (220) est positionnée au sein de l'orifice central (252) du patch (250) et tel que le boitier (120) est en contact avec la zone rigide (256) du patch (250), pour transmettre les efforts de la lanière au patch (250), afin de maintenir les aiguilles enfoncées par le biais de la capsule (220) attachée à la zone rigide (256) du corps principal (251) du patch (250).

2. Système selon la revendication 1, dans lequel la capsule (220) a une épaisseur identique à l'épaisseur de la zone rigide (256) du corps principal (501) du patch (250).

3. Système selon l'une quelconque des revendications 1 à 2, dans lequel la zone rigide (256) présente une dureté shore plus importante que la zone souple (258), par exemple une dureté shore comprise entre 20 et 40 pour la zone souple (258) et une dureté shore comprise entre 60 et 80 pour la zone rigide (256) ou bien le matériau de la zone rigide (256) est plus dur que le matériau de la zone souple (258), les deux matériaux étant différents.

4. Système selon l'une quelconque des revendications 1 à 3, dans lequel la zone rigide (256) est au moins deux fois plus épaisse que la zone souple (258), préférablement au moins trois fois plus épaisse.

5. Système selon l'une quelconque des revendications 1 à 4, comprenant en outre un adhésif plan recouvrant substantiellement une face plane des zones rigides (256) et souples (258).

6. Système selon la revendication 5, dans lequel l'adhésif (260) est à base de silicone ou à base d'acrylique.

7. Système selon l'une quelconque des revendications 1 à 6, dans lequel la zone souple (258) comprend une pluralité d'ouvertures traversantes.

8. Système selon l'une quelconque des revendications 5 à 7, dans lequel le corps principal (251), préférentiellement la zone souple (258), comprend une pluralité d'ouvertures traversantes (270), et
- l'adhésif (260) comprend une pluralité d'ouvertures traversantes,
- la taille des ouvertures (270) du corps principal (251) étant supérieure à la taille des ouvertures de l'adhésif (260), et/ou la densité de surface d'ouverture (270) du corps principal (251) est supérieure à la densité de surface d'ouverture de l'adhésif (260), et/ou la densité d'ouvertures (270) du corps principal est supérieure à la densité d'ouvertures de l'adhésif (260).

## Patentansprüche

1. System zur Körperüberwachung (1), das zur Befestigung auf einer Gliedmaße bestimmt ist, umfassend:
- ein Patch (250), das einen Hauptkörper (251) mit einer Öffnung (252) von mindestens 2 mm Durchmesser umfasst, wobei der Hauptkörper (251) einen flexiblen Bereich (258) umfasst, auf dem ein starrer Bereich (256) angebracht ist, wobei der starre Bereich (256) um die Öffnung (252) herum angeordnet ist,
- eine Kapsel (220), wobei die Kapsel (220) ausgelegt ist, um sich in der Öffnung des Patchs (250) zu platzieren und mit diesem fest verbunden zu sein, wobei die Kapsel (220) Mikronadeln (210) umfasst, die zum Einführen in die Haut ausgelegt sind, um ein Körperfluid des Trägers des Systems zur Körperüberwachung (1) zu entnehmen oder zu analysieren, wenn dieses an einer Gliedmaße eines Benutzers positioniert ist;
- ein Gehäuse (120), das imstande ist, mit der Kapsel (220) verbunden zu sein, in dessen Inneren sich eine Batterie und ein Prozessor befinden, wobei der Prozessor ausgelegt ist, um Daten zu verarbeiten, die mithilfe der Entnahme oder Messung durch die Mikronadeln (210) gewonnen wurden;
- ein Band, das ausgelegt ist, um das Gehäuse (120) an seinem Platz auf der Gliedmaße zu halten, wobei das System derart ist, dass die Kapsel (220) innerhalb der zentralen Öffnung (252) des Patchs (250) positioniert ist und derart, dass das Gehäuse (120) mit dem starren Bereich (256) des Patchs (250) in Kontakt steht, um die Kräfte des Bandes auf das Patch (250) zu übertragen, damit die Nadeln mittels der Kapsel (220), die an dem starren Bereich (256) des Hauptkörpers (251) des Patchs (250) befestigt ist, eingedrückt bleiben.

2. System nach Anspruch 1, wobei die Kapsel (220) eine Dicke aufweist, die mit der Dicke des starren Bereichs (256) des Hauptkörpers (501) des Patchs (250) identisch ist.

3. System nach einem der Ansprüche 1 bis 2, wobei der starre Bereich (256) eine höhere Shore-Härte als der flexible Bereich (258) aufweist, beispielsweise eine Shore-Härte zwischen 20 und 40 für den flexiblen Bereich (258) und eine Shore-Härte zwischen 60 und 80 für den starren Bereich (256) beziehungsweise das Material des starren Bereichs (256) härter ist als das Material des flexiblen Bereichs (258), wobei die beiden Materialien unterschiedlich sind.

4. System nach einem der Ansprüche 1 bis 3, wobei der starre Bereich (256) mindestens doppelt so dick wie der flexible Bereich (258) ist, vorzugsweise mindestens dreimal so dick.

5. System nach einem der Ansprüche 1 bis 4, das ferner einen ebenen Haftstoff umfasst, der eine ebene Fläche des starren Bereichs (256) und des flexiblen Bereichs (258) substanziell bedeckt.

6. System nach Anspruch 5, wobei der Haftstoff (260) auf Silikon- oder Acrylbasis ist.

7. System nach einem der Ansprüche 1 bis 6, wobei der flexible Bereich (258) eine Vielzahl von Durchgangsöffnungen umfasst.

8. System nach einem der Ansprüche 5 bis 7, wobei der Hauptkörper (251), vorzugsweise der flexible Bereich (258), eine Vielzahl von Durchgangsöffnungen (270) umfasst, und
- der Haftstoff (260) eine Vielzahl von Durchgangsöffnungen umfasst,
- wobei die Größe der Öffnungen (270) des Hauptkörpers (251) größer ist als die Größe der Öffnungen des Haftstoffs (260), und/oder die Oberflächendichte der Öffnungen (270) des Hauptkörpers (251) größer ist als die Oberflächendichte der Öffnungen des Haftstoffs (260), und/oder die Dichte der Öffnungen (270) des Hauptkörpers größer ist als die Dichte der Öffnungen des Haftstoffs (260).

## Claims

1. A body monitoring system (1) intended to be attached to a limb, comprising:
- a patch (250) comprising a main body (251) with an orifice (252) of at least 2 mm in diameter, the main body (251) comprising a flexible area (258) on which a rigid area (256) is arranged, the rigid area (256) being positioned around the opening (252),
- a capsule (220), the capsule (220) being configured to be placed in the opening of the patch (250) and to be secured thereto, the capsule (220) comprising microneedles (210) configured to be inserted into the skin to sample or analyze a body fluid of the wearer of a body monitoring system (1) when the latter is positioned on a limb of a user,
- a housing (120) adapted to be coupled with the capsule (220) inside which there is a battery and a processor, the processor being configured to process data obtained using the sample or the measurement performed by the microneedles (210),
- a strap, configured to hold the housing (120) in place on the limb,
the system being configured such that the capsule (220) is positioned within the central orifice (252) of the patch (250) and such that the orifice (120) is in contact with the rigid area (256) of the patch (250), so as to transmit the forces from the strap to the patch (250), thereby keeping the needles inserted through the capsule (220) attached to the rigid area (256) of the main body (251) of the patch (250.

2. The system of claim 1, wherein the capsule (220) has a thickness identical to the thickness of the rigid area (256) of the main body (501) of the patch (250).

3. The system of any one of claims 1 to 2, wherein the rigid area (256) has a Shore hardness greater than the flexible area (258), for example a Shore hardness comprised between 20 and 40 for the flexible area (258) and a Shore hardness comprised between 60 and 80 for the rigid area (256) or the material of the rigid area (256) is harder than the material of the flexible area (258), the two materials being different.

4. The system of any one of claims 1 to 3, wherein the rigid area (256) is at least twice as thick as the flexible area (258), preferably at least three times as thick.

5. The system of any one of claims 1 to 4, comprising a planar adhesive substantially covering a planar face of the rigid (256) and flexible (258) areas.

6. The system of claim 5, wherein the adhesive (260) is a silicone-based or an acrylic-based adhesive.

7. The system of any one of claims 1 to 6, wherein the flexible area (258) comprises a plurality of through openings.

8. The system of any one of claims 1 to 7, wherein
- the main body (251), preferably the flexible area (258), comprises a plurality of through openings (270), and
- the adhesive (260) comprises a plurality of through openings,
- the size of the openings (270) of the main body (251) being greater than the size of the openings of the adhesive (260), and/or the opening area density (270) of the main body (251) is greater than the opening area density of the adhesive (260), and/or the opening density (270) of the main body is greater than the opening density of the adhesive (260).
